# EUROPEAN PATENT APPLICATION

(11) **EP 4 718 375 A1**
(43) Date of publication of application: **01.04.2026**
(21) Application number: 24306605.7
(22) Date of filing: 30.09.2024
(51) Int. Cl.: G06T 7/00, A61B 6/50, G06T 7/10, G06T 11/00

(54) **SYSTEM AND METHOD FOR PROCESSING MEDICAL IMAGE DATA FOR GENERATING AN IMAGE COMPRISING A SIMULTANEOUS REPRESENTATION OF AN ARTERIAL, VALVULAR AND MYOCARDIAL STATE**

(71) Applicant: Koninklijke Philips N.V., 5656 AG Eindhoven (NL)
(72) Inventor: WIEMKER, Rafael, 5656 Eindhoven (NL); NICKISCH, Hannes, 5656 Eindhoven (NL); MATUTE FLORES, Jose Alejandro, 5656 Eindhoven (NL); HEESE, Harald, 5656 Eindhoven (NL); VILLIEN, Marjorie, 5656 Eindhoven (NL); PETERS, Jochen, 5656 Eindhoven (NL); VLACHOMITROU, Anna Sesilia, 5656 Eindhoven (NL); BONTUS, Claas, 5656 Eindhoven (NL)
(74) Representative: Philips Intellectual Property & Standards

(57) **Abstract**

The present invention relates to the field of medical imaging and in particular to a system (10) for processing medical image data for generating an image comprising a simultaneous representation of an arterial, valvular and myocardial state, wherein the system is adapted to perform the steps of obtaining 3D medical image data (S 101); identifying arterial, valvular and myocardial regions in the 3D medical image data by means of segmentation (S102); mapping the 3D medical image data to a 2D representation using a coordinate transform based on a spherical coordinate system with an origin depending on the identified valvular region and/or the identified arterial region (S104); generating a combined 2D image (50) indicative of the arterial-, valvular and myocardial state based on the 2D representation as an output (S104). The present invention further relates to a corresponding method (100) and computer program.

## Description

### FIELD OF THE INVENTION

The present invention relates to the field of medical imaging systems, and in particular to a system for processing medical image data for generating an image comprising a simultaneous representation of an arterial, valvular and myocardial state. The present invention furthermore relates to a corresponding method as well as to a corresponding computer program for carrying out said method.

### BACKGROUND OF THE INVENTION

Cardiac CT, or cardiac computed tomography, is an advanced medical imaging technique used to obtain detailed pictures of the heart and its blood vessels. This non-invasive procedure utilizes X-ray technology combined with computer processing to create high-resolution, cross-sectional images of the heart. It is particularly valuable for assessing coronary artery disease (CAD), evaluating the anatomy of the heart and surrounding structures, and planning interventions such as stent placements or bypass surgery. The excellent spatial as well as dynamic and spectral resolution of CT and in particular Photon Counting CT allows highly detailed anatomical and functional contrast visualization. During a cardiac CT scan, a contrast dye or contrast/perfusion agent can optionally be injected into a blood vessel such as a vein to enhance the visibility of blood vessels and heart chambers, providing clear images of any blockages, plaque buildup, or other abnormalities. Cardiac CT is advantageous for its precision, allowing for early detection and treatment planning of heart conditions, thereby improving patient outcomes. It is a critical tool for cardiologists to diagnose and manage heart disease, offering a safer alternative to invasive procedures like coronary angiography. The detailed 3D images produced by cardiac CT help in better understanding of heart diseases, facilitating more accurate diagnoses and targeted treatments.

The obtained 3D medical image data is usually reviewed on high-power workstations with fast data connections and dedicated graphics chip sets, wherein an operator such as a radiologist quickly scrolls through different reconstructed layers. The operator carefully adjusts different scan windows for sequentially reviewing structures of different densities. This can be a time-consuming process and requires costly processing equipment with high computational power for each reviewer.

### SUMMARY OF THE INVENTION

It is an object of the present invention to overcome technical limitations of existing workstations for reviewing 3D medical images and to provide a further improved system that assists a user in reviewing 3D medical image data. In particular, it would be desirable to facilitate sharing and enable reviewing 3D medical image data on devices with limited connection speed and limited processing power in a user-friendly and understandable manner. Moreover, it would be desirable to reduce the complexity when handling 3D medical image data.

In a first aspect of the present invention a system for processing medical image data for generating an image comprising a simultaneous representation of an arterial, valvular and myocardial state, is presented, wherein the system is adapted to perform the steps of:
- obtaining 3D medical image data;
- identifying arterial, valvular and myocardial regions in the 3D medical image by means of segmentation;
- mapping the 3D medical image data to a 2D representation using a coordinate transform based on a spherical coordinate system with an origin depending on the identified valvular region and/or the identified arterial region;
- generating a combined 2D image indicative of the arterial-, valvular and myocardial state based on the 2D representation as an output.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium can have similar and/or identical preferred embodiments as the claimed system, in particular as defined in the dependent claims and as disclosed herein.

The present invention is based on the idea to overcome technical limitations of existing workstations for reviewing 3D medical images and to facilitate the sharing and reviewing of 3D medical images. It is suggested to provide a system for automatically generating a representation of a volume of interest in a 3D medical image wherein the representation provides a one-glance overview for multiple clinical problems. It becomes possible to capture the status with respect to the trinity of the three most examined cardiac diseases, namely arterial (e.g. indicative regarding state of coronaries), valvular (e.g. indicative regarding valve replacement), myocardial/muscular (e.g. indicative regarding perfusion), in a single synoptic view. Advantageously, possible interactions between arterial stenoses and myocardial perfusion defects can become visible. By providing a 2D or "flat" image based on the proposed transformation, the resulting combined 2D image - while still being indicative of the arterial-, valvular and myocardial state - is highly reproducible, e.g. displayable/stackable for dashboard purposes and printable. The combined 2D image can also easily be shared even over a slow connection and reviewed on a device with limited processing power.

Moreover, by providing a static or non-interactive representation, even a mobile device without advanced HMI interface for handling 3D objects can be sufficient. Thereby, the 3D medical image data is processed in a specific manner for review not only on dedicated radiology workstations with respective input means for scrolling through image sets or manipulating 3D renderings.

A further advantage can be that the proposed solution can be implemented via an analytical algorithm with the proposed transform, such that no manual or AI-based segmentation of the coronaries may be required. Thereby a reliable and reproducible output can be provided.

The step of generating the combined 2D image indicative of the arterial-, valvular and myocardial state can comprise combining respective three input sources respectively indicative of the arterial state, of the valvular state and of the myocardial state for one or more, in particular for each map pixel of the combined 2D image. Generating the combined 2D image can comprise combining separate 2D images indicative of the arterial, valvular and myocardial state. This can involve (a) identifying one or more arterial regions in the 3D medical image data by means of segmentation, mapping the 3D medical image data to a 2D representation of the arterial regions, and generating a 2D image indicative of the arterial state based on the 2D representation of the arterial regions; (b) identifying one or more valvular regions in the 3D medical image data by means of segmentation, mapping the 3D medical image data to a 2D representation of the valvular regions, and generating a 2D image indicative of the valvular state based on the 2D representation of the valvular regions; and (c) identifying one or more myocardial regions in the 3D medical image data by means of segmentation, mapping the 3D medical image data to a 2D representation of the myocardial regions, and generating a 2D image indicative of the myocardial state based on the 2D representation of the myocardial regions. The different images can then be combined to generate the combined 2D image indicative of the arterial-, valvular and myocardial state based on the 2D representation as an output.

As used herein, "obtaining" 3D medical image data can also refer to receiving or retrieving medical image data, for example from a storage such as a database. In other words, an initial acquisition of the 3D medical image data can be performed by a medical imaging modality such as a CT or MRI device. The 3D medical image data can either directly be provided to the system for processing the medical image data or can be buffered or stored for subsequent processing.

The system for processing medical image data can be implemented in different forms, including but not limited to a workstation, a server, a distributed processing system, or a cloud-based system.

In an embodiment, the mapping of the 3D medical image data to a 2D representation can be based on a spherical coordinate transform with an origin at a ventricle, in particular with an origin at a centroid of the left ventricle. An advantage of this refinement can be that a particularly advantageous one-glance overview for multiple clinical problems can be provided.

In a further refinement, the spherical coordinate transform can be performed with a z-normal towards the aortic valve for generating a valvular view. In addition or in the alternative, the spherical coordinate transform is performed with a z-normal towards a ventricular apex, in particular towards the left ventricular apex, for generating and apical view. An advantage of this refinement can be that such a defined orientation facilitates image review by a clinical practitioner. Moreover, a correspondence with SCCT, AHA diagrams can be provided, which can facilitate image review and comparison. SCCT and AHA diagrams can refer to representations suggested by the SCCT, Society of Cardiovascular Computed Tomography, and also regarding CCTA, Coronary Computed Tomography Angiography.

It should be noted that in a further refinement, both views can be generated. In other words, the system can be adapted to perform (a) a first mapping of the 3D medical image data to a first 2D representation using a first coordinate transform based on a spherical coordinate system with an origin depending on the identified valvular region, in particular wherein the spherical coordinate transform is performed with a z-normal towards the aortic valve for generating a valvular view; and generating a first combined 2D image part indicative of the arterial-, valvular and myocardial state based on the first 2D representation; and (b) a second mapping of the 3D medical image data to a second 2D representation using a second coordinate transform based on a spherical coordinate system with an origin depending on the identified arterial region, in particular wherein the spherical coordinate transform is performed with a z-normal towards the left ventricular apex for generating an apical view; and generating a second combined 2D image part indicative of the arterial-, valvular and myocardial state based on the second 2D representation. Both 2D image parts can then be provided as a combined 2D image, for example next to each other.

A volume of interest around the segmented valvular region can be mapped using the coordinate transform and using an intensity projection, in particular wherein for the valvular region a mean intensity projection can be used. Thereby tri-cuspid or bicuspid valvular leaflets and raphes, etc. can be shown advantageously.

In addition or in the alternative, 3D voxels of the segmented arteries of an arterial region and/or 3D voxels of the segmented myocardial region can be mapped using the coordinate transform and using an intensity projection.

In a further refinement, for the arterial region a maximum intensity projection can be used. In other words, 3D voxels with high coronary likelihood can be mapped using the coordinate transform and an intensity projection in particular a maximum intensity projection. This can facilitate showing a contrast agent uptake and can assist in highlighting also high opacities calcifications, stents, etc..

In addition or in the alternative, for the myocardial region a mean or median intensity projection can be used. In other words, 3D voxels belonging to the myocardium can be mapped using the coordinate transform and an intensity projection, in particular a median intensity projection. This can help to minimize an influence of fringe voxels from delineation inaccuracies. In a further refinement, values and respective pixels indicative of a myocardial region can be pseudo-color encoded relative to the median of the overall myocardium. The system can be adapted to perform the steps of: determining a global median intensity of the myocardial region, e.g. a global median of an overall myocardium of the myocardial region; determining a local median intensity for pixels of the 2D representation based on a median intensity projection; determining differences between the local median intensity and the global median for the pixels of the 2D representation; and generating a color-coded 2D representation indicative of the myocardial state based on the determined differences for the pixels of the 2D representation. In other words, the local median values can be pseudo-color encoded (positive / negative) relative to the global median of the overall myocardium. An advantage can be that under- and over-perfusion of the myocardium can be indicated in intuitively perceivable spatial context of the depicted coronaries. The proposed system thereby further assists a practitioner in reviewing potentially relevant deviations.

It will be appreciated that that one or more of the above can be combined. In an advantageous embodiment, a combined 2D image indicative of the arterial-, valvular and myocardial state can be provided, wherein for the for the arterial region a maximum intensity projection is used indicative of the arterial state, for the valvular region a mean intensity projection is used indicative of the valvular state, and for the myocardial region a median intensity projection is used indicative of the myocardial state. Hence, a novel approach is presented wherein the system is adapted to provide a combined 2D image, wherein max-, mean- and median-projection are all specifically adapted for the different anatomical areas of interest.

For one or more of the pixels of the combined 2D image, a pointer to a corresponding location in the 3D medical image data can be stored. Thereby a correspondence between the combined 2D image and the initial 3D medical image data can be established. For example, a 3D coordinate can be stored for one or more, in particular for each, pixel pointing to the original location of the most salient composite source. This can allow navigating to the corresponding point of interest in the 3D image volume, e.g., CT scan or MRI scan. Hence, in addition to facilitating a diagnostic overview, the combined 2D image, also referred to as key image, can be used for quick navigation to locations of interest, for example in a standard slice-wise viewer. For example, a mouse click on key-image locations bringing up the corresponding image slice with a marker.

Mapping the 3D medical image data can comprise: mapping the 3D medical image data to a first 2D representation using a first coordinate transform based on a first spherical coordinate system with origin depending on the identified valvular region; and mapping the 3D medical image data to a second 2D representation using a second coordinate transform based on a second spherical coordinate system with origin depending on the identified arterial region; wherein generating the combined 2D image indicative of the arterial-, valvular and myocardial state comprises generating a first combined image part based on the first 2D representation and a second combined image part the second 2D representation. Thereby, an advantageous mapping regarding two different spherical coordinate systems can be provided. This allows the user to capture a cardiac state of a patient very efficiently while at the same time enabling image review on a device with limited computational power.

In a refinement, the first combined image part and the second combined image part can be arranged in a single combined 2D image indicative of the arterial-, valvular and myocardial state. In particular, the first combined image part and the second combined image part can be arranged next to each other. Thereby, the proposed system further facilitates assessment of a cardiac state in a well-defined manner while at the same time enabling image review on a device with limited computational power.

The system can further be adapted to perform the step of estimating myocardial feeding territories and generating the combined 2D image further comprising the estimated myocardial feeding territories. An advantage of this embodiment can be that review of medical image data is further facilitated by the proposed system. Moreover, the combined 2D image can be easily shared and reviewed also on devices with a slow connection and/or with limited processing power.

The system can further be adapted to perform the step of mapping by means of a step of interactively adjusting a focus direction vector and/or a zoom factor using an interactive user interface. The mapping depends on the adjusted focus direction vector and/or the zoom factor.

This allows for an efficient appraisal, marking and quantification of coronary locations in conjunction with myocardial areas, which is necessary for differential diagnosis of coronary artery disease (CAD). It further allows for a possibility to absolve `full' comprehensive reading and annotation on a 'flat' 2D representation without loss of spatial resolution nor dynamic fidelity (spectral or Hounsfield units in case of CT), by allowing focus and zoom selection for local inspection. It advantageously avoids being dependent on error-prone coronary centerline estimation and may save reading and/or calculation time in comparison to per-segment vessel stretching and rotation.

It further contributes to exploitation of superior spatial and spectral sensitivity of photon counting CT over conventional CT.

In other words, adjusting and/or selecting the focus direction vector and/or a zoom factor by means of an interactive user interface allows for a mapping that contributes to an improved exploitation of advanced spatial resolution of photon counting CT.

The system is further adapted to perform the step of mapping by means of a step of interactively adjusting an intersection depth using an interactive user interface. This allows adjustment of an intersection depth offset radially to the origin, wherein the offset is global and relative to a locally variable absolute 3D vector. The mapping depends on the adjusted intersection depth.

Surprisingly, also this allows for an efficient appraisal, marking and quantification of coronary locations in conjunction with myocardial areas, which is necessary for differential diagnosis of coronary artery disease (CAD). It allows for a possibility to absolve `full' comprehensive reading and annotation on a 'flat' 2D representation without loss of spatial resolution nor dynamic fidelity (spectral or Hounsfield units in case of CT), by allowing intersection depth selection for local inspection. It advantageously avoids being dependent on error-prone coronary centerline estimation and may save reading and/or calculation time in comparison to per-segment vessel stretching and rotation.

It further contributes to exploitation of superior spatial and spectral sensitivity of photon counting CT over conventional CT.

In other words, adjusting and/or selecting the intersection depth by means of an interactive user interface allows for a mapping that contributes to an improved exploitation of advanced spatial resolution of photon counting CT.

It is particularly advantageous to allow adaptation of the focus direction vector, the zoom factor and the intersection depth by means of the interactive user interface. Allowing selection and adaptation of these three properties has as advantage that it allows for revealing all relevant anomalies of interest (stenoses, plaques, calcifications, perfusion defects, etc.) in a 2D combined image even when those anomalies of interest are embedded or buried in a 3D image with high and complex spatial, dynamic and spectral resolution.

It shall be understood that the system can optionally also include the actual image acquisition device such as a CT or MRI scanner. However, it is also possible to use the proposed system in combination with an existing image acquisition device and/or with a data storage providing the 3D medical image data as an input to the proposed system according to aspects of the present disclosure.

### BRIEF DESCRIPTION OF THE DRAWINGS

These and other aspects of the invention will be apparent from and elucidated with reference to the embodiment(s) described hereinafter. In the following drawings
Fig. 1 shows a schematic block diagram of a system for processing medical image data according to an aspect of the present disclosure;
Fig.2 shows a flow chart of a method for processing medical image data according to an aspect of the present disclosure;
Fig. 3 shows a first exemplary image based on a valvular centric mapping;
Fig. 4 shows a second exemplary image based on an apical centric mapping;
Fig. 5 shows a third exemplary image with two views;
Fig. 6 shows a fourth exemplary image with two views;
Fig. 7 shows a fifth exemplary image with coronary feeding territory;
Fig. 8 shows a sixth exemplary image with coronary feeding territory;
Fig. 9 shows an illustration illustrating a computation of connectivity between coronaries;
Fig. 10 shows a projection geometry;
Fig. 11 shows an example of a combination view that is adjustable and/or selectable in terms of focus direction vector, zoom factor and/or intersection depth;
Fig. 12 shows an example of a view with an adjusted zoom factor;
Fig. 13 shows an example of a view with an adjusted focus direction vector; and
Fig.14a, 14b, 14c and 14d show an example of a view with adjusted intersection depths.

### DETAILED DESCRIPTION OF THE INVENTION

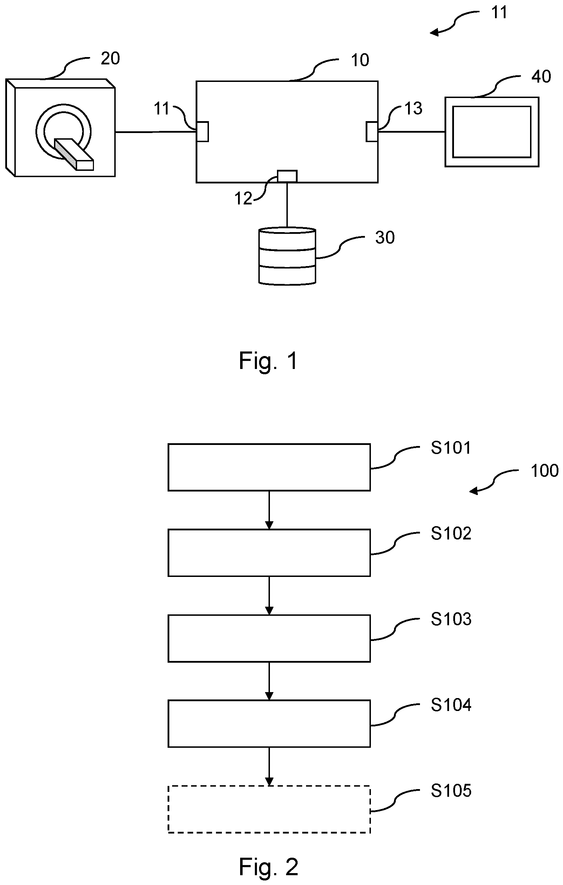

Fig. 1 shows an embodiment of a system 10 for processing medical image data. The system 10 is adapted for generating an image comprising a simultaneous representation of an arterial, valvular and myocardial state. A corresponding method 100 is illustrated in the flow chart in Fig. 2.

As shown in Fig. 1, an image acquisition device 20 such as a CT scanner or MRI scanner can be provided for acquisition of 3D medical images. The acquired 3D medical images are then provided to the system 10 via a communication interface 11. The communication interface 11 can be a wired or wireless communication interface. The excellent spatial as well as dynamic and spectral resolution of CT and in particular Photon Counting CT scanners allows highly detailed anatomical and functional (contrast) visualization for diagnostic appraisal. It shall be understood that the 3D medical image data is not limited to CT image data but can also be acquired using different modalities. The teachings can in the same way be applied to conventional, spectral, and photon-counting CT. The teachings can in the same way be applied to other cardiac 3D modalities, e.g., MRI or 3D ultrasound.

In addition or in the alternative to directly obtaining the 3D medical image data from an image acquisition device 20, the 3D medical image data can also be provided from a storage 30 such as a database. The 3D medical images can be provided to the system 10 via a communication interface 12. The communication interface 12 can again be a wired or wireless communication interface. The 3D medical image data can thus either directly be provided to the system 10 for processing the medical image data or can be buffered or stored for subsequent processing. More generally speaking, the system 10 is thus adapted for obtaining 3D medical image data.

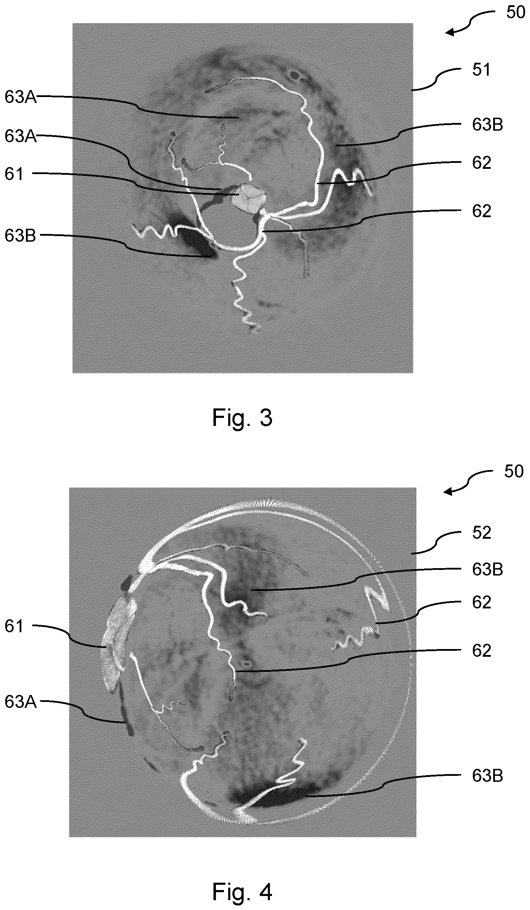

The system 10 of Fig. 1 is adapted to perform the steps of the method 100 of Fig. 2. Exemplary output images comprising a simultaneous representation of an arterial, valvular and myocardial state according to certain aspects of the present disclosure are shown in Fig. 3 to Fig. 8.

In a first step S101, 3D medical image data is obtained. As described above, the 3D medical image data can be obtained from an image acquisition device 20 and/or from a storage 30.

In a second step S102 arterial, valvular and myocardial regions in the 3D medical image data are identified. The different regions can be identified by means of segmentation. For example, the system 10 can be configured for automatic detection and delineation of a cardiac VOI (volume of interest), a myocardium such as a myocardium surrounding the left ventricle, an aortic valve, and a blood vessel such as an ascending aorta. All these components are uncritical to detect and delineate automatically as they are known to be present, to manifest in exactly one instance each, and to be contained in rather compact volumes of interest. However, the present disclosure is not limited thereto.

In a third step S103 the 3D medical image data is mapped to a 2D representation using a coordinate transform based on a spherical coordinate system. As used herein the mapping of the 3D medical image date to a 2D representation can refer to or be based on a polar transform. An origin for the mapping can depend on the identified valvular region and/or the identified arterial region. A distance from the coordinate original may be neglected.

For example using pseudo-code a 3D to 2D mapping can be based on a polar transform, neglecting the distance from the coordinate origin:

```
// Calculate the difference vector between point pMM and the origin
 var diffVec = (pMM - coo.origin);
 // Compute the angle theta between the normalized difference vector and the ez vector of coo
 var theta = Math.Acos(diffVec.Normalized() * coo.ez) / Math.PI; // Normalized to range 0..1
 // Determine the radial distance based on theta
 var radialDistance = theta * (width / 2 - 1);
 // Project the difference vector onto the ex and
ey axes of the coordinate system and normalize it
 var projectedDifference = new DoublePoint2D(difference
* coo.ex, difference * coo.ey).Normalized();
 // Map the projected and normalized coordinates to an integer point within the image space
 var mappedPoint = new IntPoint2D(
  width / 2 + projectedDifference.x * radialDistance,
  width / 2 + projectedDifference.y * radialDistance
 );
```

In a fourth step S104, a combined 2D image indicative of the arterial-, valvular and myocardial state based on the 2D representation is generated as an output.

Referring again to Fig. 1, an output device 40 is provided that can be coupled with the system 10 for processing the medical image data for generating an image comprising a simultaneous representation of an arterial, valvular and myocardial state. The output device 10 allows a user such as a medical practitioner to review the combined 2D image indicative of the arterial-, valvular and myocardial state based on the 2D representation as the output. The output device 40 can be a device with a screen for reviewing the combined 2D image but may have a limited connection speed and limited processing power. For example, the output device 40 can be a mobile device such as a tablet or even a smartphone. The specific image processing as suggested herein also overcome the need from complex HMI (human machine interface) interactions such as windowing based on different Hounsfield densities or scrolling through different layers of slice-wise 3D medical imaging data.

The overall system comprising the system 10 for processing the medical image data as well as the image acquisition device 20 and/or the storage 30 and optionally the output device 40 may be denoted by reference numeral 11. It is also possible that the image acquisition device 20 and/or the storage 30 can be part of the system 10.

The mapping of the 3D medical image data to a 2D representation, as exemplarily shown in step S102, can be based on a spherical coordinate transform with an origin at a ventricle, in particular with an origin at a centroid of the left ventricle. The spherical coordinate transform can be performed with a z-normal towards the aortic valve for generating a valvular view. A corresponding resulting combined 2D image indicative of the arterial-, valvular and myocardial state based on the 2D representation is exemplarily shown in Fig. 3. Accordingly, Fig. 3 shows a first exemplary image based on a valvular centric mapping.

In addition or in the alternative ,the mapping of the 3D medical image data to a 2D representation, as exemplarily shown in step S102, can be based on a spherical coordinate transform with a z-normal towards a ventricular apex, in particular towards the left ventricular apex, for generating and apical view. A corresponding resulting combined 2D image indicative of the arterial-, valvular and myocardial state based on the 2D representation is exemplarily shown in Fig. 4. Accordingly, Fig. 4 shows a second exemplary image based on an apical centric mapping.

It should be noted that the two mappings, e.g. the mapping regarding the aortic valve as exemplarily shown in Fig. 3 and the mapping regarding the left ventricular apex as exemplarily shown in Fig. 4, are not necessarily exact oppositional (180 deg) but can be oblique to each other. Preferably, the x-normals of the projections can be rotated such as to maximize correspondence with SCCT, AHA diagrams, respectively. This can facilitate image review by a medical practitioner.

Referring again to Fig. 2, Step S102, step S103 and/or step S104 can comprise several sub-steps that can be performed sequentially or in parallel.

For example, step S102 can comprise one or more of a first sub-step S102A of identifying one or more arterial regions in the 3D medical image data by means of segmentation, a second sub-step S102B of identifying one or more valvular regions in the 3D medical image data by means of segmentation, and a third sub-step S102C of identifying one or more myocardial regions in the 3D medical image data by means of segmentation.

For example, step S103 can comprise one or more of a first sub-step of S 103A, a second sub-step S 103B and a third sub-step S103C. Sub-step S103A can comprise mapping the 3D medical image data to a 2D representation of the arterial regions, and generating a 2D image indicative of the arterial state based on the 2D representation of the arterial regions. For example, 3D voxels in the 3D medical image date with high coronary likelihood can be mapped using the coordinate transform and preferably a max intensity projection. This can facilitate showing a contrast agent uptake and can assist in highlighting also high opacities calcifications, stents, etc.. Sub-step S 103B can comprise of mapping the 3D medical image data to a 2D representation of the valvular regions, and generating a 2D image indicative of the valvular state based on the 2D representation of the valvular regions. For example, the volume of interest (VOI) around the aortic valve is mapped using the coordinate transform and preferably a mean intensity projection. Thereby tri-cuspid or bicuspid valvular leaflets and raphes, etc. can be shown advantageously. Sub-step S103C can comprise mapping the 3D medical image data to a 2D representation of the myocardial regions, and generating a 2D image indicative of the myocardial state based on the 2D representation of the myocardial regions. For example 3D voxels, preferably all 3D voxels, belonging to the myocardium are mapped using the coordinate transform and preferably a median intensity projection. This can help to minimize an influence of fringe voxels from delineation inaccuracies. In a further refinement, values and respective pixels indicative of a myocardial region can be pseudo-color encoded relative to the median of the overall myocardium. Preferably, the values are pseudo-color encoded relative to the median of the overall myocardium.

In step S104, the different images can then be combined to generate the combined 2D image indicative of the arterial-, valvular and myocardial state based on the 2D representation as an output. In other words, step S104 can be seen as a compositing step combining the three input sources for each map pixel or to provide a combined 2D image indicative of the arterial-, valvular and myocardial state based on the respective 2D representations. In other words, several separate 2D representations can be generated. Each involves mapping the 3D medical image data to a 2D representation using a coordinate transform based on a spherical coordinate system with an origin depending on the identified valvular region and/or the identified arterial region. A first 2D representation indicative of the arterial state, a second 2D representation indicative of the valvular state and a third 2D representation indicative of the myocardial state. The first, second and third 2D representation are then combined to generate a combined 2D image indicative of the arterial-, valvular and myocardial state based on the 2D representations as an output in compositing step S104.

An optional step S105 comprises navigating to an original location in the 3D medical image date based on a selected location in the combined 2D image. For example, a 3D coordinate can be stored for one or more, in particular for each, pixel pointing to the original location of the most salient composite source. This can allow navigating to the corresponding point of interest in the 3D image volume, e.g., CT scan or MRI scan.

Referring again to Fig. 3 and Fig. 4, both images are based on the same 3D medical image data. Fig. 3 shows a first exemplary combined 2D image 50 based on a valvular centric mapping 51 and Fig. 4 shows a second exemplary combined 2D image 50 based on an apical centric mapping. The respective combined 2D images 50 are indicative of the indicative of the arterial-, valvular and myocardial state. In the combined 2D image the heart valve 61, the arteries 62 as well as the myocardial region 63.

In the shown example, the way that the difference structures are presented is based on the specific image data processing as described herein. For example, image components of the heart valve 61 is determined by mapping a volume of interest around the aortic valve using the coordinate transform and a mean intensity projection. The image components of the arteries 62 is determined by mapping 3D voxels in the 3D medical image data with high coronary likelihood using the coordinate transform and preferably a maximum intensity projection. The image components of the myocardial region 63 are determined by mapping 3D voxels belonging to the myocardium using the coordinate transform and a median intensity projection. Advantageously, the myocardial region 63 can be provided using color coding wherein regions 63B below a median value are shown e.g. in blue and regions 62A above a median value are shown in red. More precisely the system can be adapted to determine a global median of an overall myocardium of the myocardial region. Hence, 3D voxels of the 3D medical image which are attributed to the myocardial region can be determined and a global median value of these 3D voxels can be determined. Further, a local median intensity for pixels of the 2D representation based on a median intensity projection. Hence, the 3D voxels of the 3D medical image that are projected onto a 2D pixel of the 2D image representation are evaluated and a median intensity of these 3D voxels is used for determining the local value of the corresponding pixel. For each of said 2D pixels, the value of the local median intensity projection for that pixel can be compared with the global median value. The differences between the respective local median intensity and the global median for the pixels of the 2D representation can be determined. Based thereon a color-coded 2D representation indicative of the myocardial state can be determined. The local median values can be pseudo-color encoded (positive / negative) relative to the global median of the overall myocardium. This allows a clinical practitioner to quickly capture the status with respect to the trinity of the three most examined cardiac diseases, namely arterial (e.g. indicative regarding state of coronaries), valvular (e.g. indicative regarding valve replacement), myocardial/muscular (e.g. indicative regarding perfusion), in a single synoptic view.

Fig. 3 provides a valvular centric map that emphasizes in particular the proximal parts of the coronary arteries 62. On the other hand, Fig. 4 provides an apical-centric map that emphasizes in particular the distal parts of the coronaries.

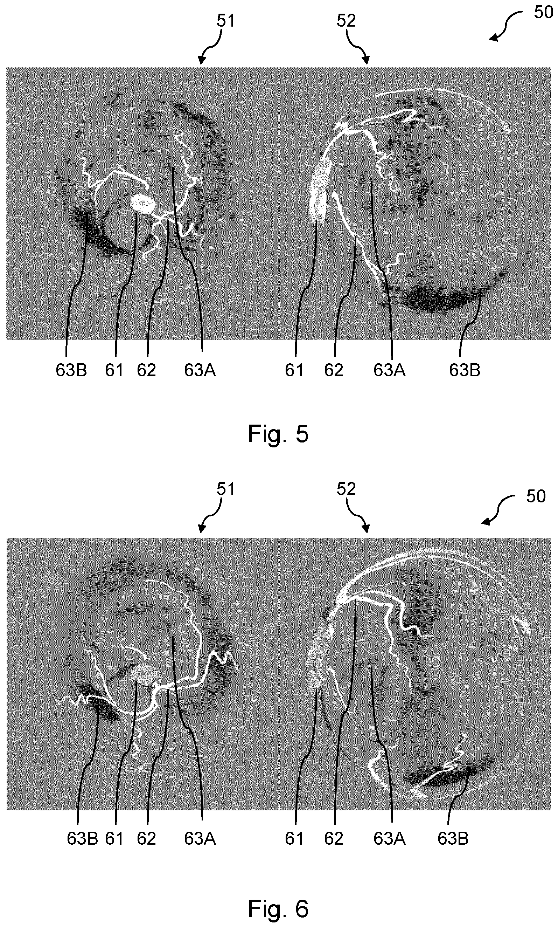

In an embodiment, it is also possible to combine two different views in a single combined image. This is exemplarily shown in Fig. 5 and Fig. 6. Accordingly, Fig. 5 and Fig. 6 show a third and fourth exemplary combined 2D image 50 with two views 51, 52 indicative of the arterial-, valvular and myocardial state based on the 2D representations of the heart valve 61, the arteries 62, and the myocardial region 63. 63A, 63B.

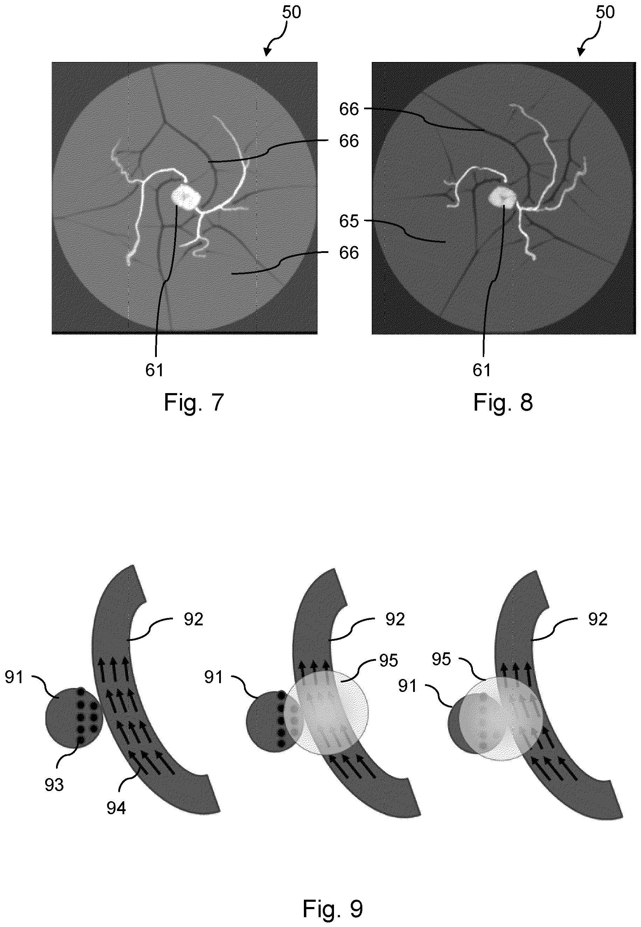

Optionally, the system is further adapted for estimating myocardial feeding territories and generating the combined 2D image 50 further comprising the estimated myocardial feeding territories 65. For example, the system can be adapted for estimating myocardial feeding territories from coronary likelihood values, for example as propagated in a voxelwise fashion from the aorta. The borders of the feeding territories 66 may be overlayed into the polar projection, es exemplarily indicated in Fig. 7 and Fig. 8. It should be noted that a feeding territory (FT) can be defined individually for each root on the coronaries, yielding a multitude of territories not statically displayable on a single diagram. In the exemplary embodiment shown herein, a 'border' of an FT can be marked at all locations where vectors of a dense FT vector field (2D, on the combined image) may be showing a negative vector divergence.

Fig. 9 shows an illustration illustrating a computation of connectivity between coronaries 91, 92. A voxelwise coronary likelihood can, e.g., be computed for example by tensor-alignment-connectivity. The connectivity can be propagated e.g. from at least two sources of seeds: an ascending aorta (as `positive` seeds), and the boundary of the volume of interest ('negative' seeds, connecting to outside structures not of interest). A prioritized region-growing can determine for each voxel the connectivity (minimum of maximizing path), where connectivity-paths are competing between `positive` and 'negative'-interest seeds. In the rendering, only voxels with `positive -interest path-connectivity are considered. The local connectivity can be determined by quantifying a scalar alignment value of local direction estimates 93, 94 of neighboring voxels as exemplarily indicated in Fig. 9.

For efficient computation, a reformat can be computed of a bounding box around the cardiac VOI, using an isotropic resampling of the VOI, to save memory and to normalize spatial derivatives in x, y, z. The local direction can be estimated weakly by computing a local Hessian matrix for each voxel, efficiently stored as three gradient volumes and six independent second-derivative volumes. A local direction vector can be adopted as the eigenvector (unit length) of highest absolute eigenvalue magnitude, multiplied by the square root of the corresponding eigenvalue. A local directional alignment can be estimated using a local Structure Tensor, defined by an outer vector product of the local direction vector, yielding six independent components per voxel. All six components can be locally accumulated in a weighted neighborhood 95 by using a standard 3D Gaussian smoothing-filter applied to the tensor volume formed by all voxel-wise structure tensors. The scalar alignment value can then be derived from the largest eigenvalue. The connectivity from the seeds to each voxel can be established efficiently using a prioritized region-growing (max-score-/min-cost-path propagation, implemented using e.g., parallelized relaxed priority queues).

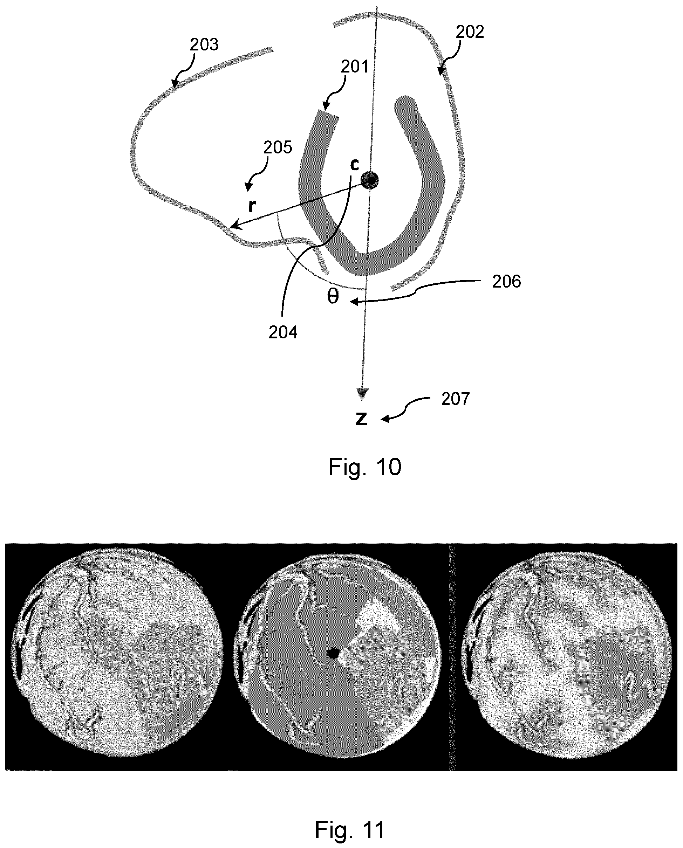

Fig. 10 discloses a projection geometry according to an embodiment of the invention. Shown is a schematic representation of a left myocardium 201, a right coronary 202 and a left coronary 203. In this cross-sectional view, a left ventricle centroid c 204 is shown to be situated inside the left myocardium 201.

A radius ***r*** 205 (or radial distance) indicates a local radial vector originating from the left ventricle centroid 204 pointing towards the left- and right coronaries and represented in polar coordinates. The local radial vector indicates a distance between the left ventricle centroid 204 and one of the left- and right coronaries under an angle *θ* 206 with respect to a focus direction of a normal vector ***z*** 207 that originates from the left ventricle centroid.

In an embodiment, the 3D to 2D (geometric) mapping is based on a polar transform similar to earlier described embodiments, neglecting the distance |**r**| from the coordinate origin **c.** Each 3D coordinate **x** is assigned a radial vector **r = x - c** and an angle *θ* = arccos(**n·r**), wherein its range: [0..*π*] is mapped to map center towards map margin). An angle **Φ** = arccos(**s·r**), wherein its range is [-*π*..+*π*] and wherein s is in the direction of the interventricular septum.

The (geometric) mapping may be similar to the previous embodiments. In addition, the mapping now depends on a focus direction vector and/or a zoom factor that are adjustable and/or selectable by means of an interactive user interface. Also, an intersection depth may be adaptable by means of the interactive user interface in combination with the focus direction vector and/or the zoom factor or on its own.

In this embodiment, all (discrete, sparse) 3D voxel locations ***x*** are mapped onto 2D pixel locations ***m*** by the currently designated geometric transformation (normal direction, zoom factor, relative radial distance). At each pixel ***m,*** the coordinate ***x*** and the weight *w* is stored.

In the map, all so far unset (undefined) pixels ***m*** which are close (less than a certain map distance away) to the sparsely set pixels, are inter-/extrapolated, yielding interpolated coordinates ***x*(*m*)** and weights *w*(***m***).

This can be computed efficiently by a Gaussian smoothing (over the 2D map plane) of G<wx>,G<wy>,G<wz>,G<w>, where *{x,y,z}* are the components of the 3D coordinate ***x*,** followed by a component-wise division ***x*** = [ <wx>, *<wy>, <wz>* ] / <w>, for all locations ***m*** where <w> is not vanishing.

For all set pixels ***m*** in the map, the 3D voxel location ***x*** is sampled in the original 3D image volume grid, using 3D interpolation (e.g., trilinear, or cubic splines). The sampled value is than depicted at its map position ***m.***

Before sampling (3), the location ***x*** can be offset with a relative radial displacement *Δr* along the normalized radial direction vector ***z*** (originating from the ventricle centroid ***c***): **x'** = **x** + Δr ***z***.

Alternatively, a line segment, i.e., a range of a certain thickness Δr is sampled yielding a series of image intensities, and the mean, median, minimum, maximum or quantile value is computed from this value list, and selected for depiction at map position ***m.***

The samples can be taken from the conventional CT image volume, a virtual mono-energy image, or a spectral map such as Iodine-, Electron-Density.

For all yet unset (blank or with vanishing weight ***w***) pixels ***m*** in the map, the 3D location ***x*** on the myocardial manifold is determined. This is done by a ray-intersection starting at the ventricle centroid c and a normalized ray vector ***z*** which is given by the map position and the current mapping geometry.

The intersection with the myocardium (described e.g., as a triangle mesh) yields an entering and a leaving point. For this liner segment, a value is derived depending on the user choice, e.g., the median or mean HU-value of the conventional CT image or a virtual mono-energy image, or another spectral map such as Iodine- or Electron-Density. The sampled value is then depicted as a gray value or color-mapped at the map position ***m.***

Figure 11 shows an example of a combination view which is user selectable by means of an interactive user interface.

In the image on the left coronaries (Hounsfield units, HU, showing stent and calcifications) are shown together with total-median-normalized myocardial local transmural median. In this publication, the image is in grayscale. However, typically, hypo areas are shown in blue, hyper areas are shown in red and user-seed-selected computed feeding territory estimates (LCX) are shown in a different color, oftentimes in yellow.

In the center image coronaries together with standard myocardial segments as defined by the American Heart Association (AHA) are show, for segmental reporting.

In the right image coronaries together with computed feeding territory estimates are shown. In this publication, the image is in grayscale. However, typically, a low nearest Euclidean myocardial-surface-distance is disclosed in red, a high nearest Euclidean distance is disclosed in blue and a user-seed-selected area (LCX) is disclosed in another color, typically yellow.

In particular, a projection of the radio-densities (HU values) of the coronaries is achieved in the following way, allowing interactively fast rendering updates:
A 'splashing' of the 3D-positions of the coronary voxel set onto the 2D map, yielding a sparsely populated 2D-map of 3D-positions with weights (4D-vector). The sparse locations of 4D vectors are padded by an interpolation scheme (e.g., component wise Gaussian smoothing or cubic- or thin-plate-splines). The radio-densities in the map are filled in by sampling the original 3D image volume at the 3D-positions provided by the padded map. This allows to retain the original spatial resolution without compromising by the (geometrically distorting) splatting process (see also embodiment above).

Preserving the high spatial 3D resolution of, e.g., photon-counting CT scanners for 'full' reading into zoomable flat 2D, the rendering of the sparse coronaries on a dense (but support-limited) background (the myocardial muscle) is made possible by implementing (a) forward projection of voxel coordinates 3D → 2D (splashing), (b) interpolation of sparse splashed points in 2D, (c) back projection of 2D-interpolated coordinates to 3D, and (d) interpolating value resampling in 3D (e.g. tri-linear or cubic-spline interpolation between eight nearest voxels).

In an embodiment, the projection of the myocardium across the myocardial wall is computed on a sampling ray cast out radially from the center of the left ventricle, and can be switched between e.g., mean/median/quantile intensity values, or trans-mural rations, or trans-mural differences. The densities can be displayed as gray values or pseudo-color palettes, in absolute or median-relative values (hyper- vs hypo-densities).
The projection of the myocardium can be switched between local properties such as: the AHA-segment number, local myocardial wall thickness, etc.

In an embodiment a discriminative visualization is applied. Since the coronaries may have asymmetrical stenoses (which would require three-dimensional `roll-around' inspection), and known to be medically significant only with a severity of more than 50%, they are depicted using a quantile-intensity-projection (say, 50% aka median, user-adjustable) which cue coronary defects (without oversight by e.g., Mean- or Maximum-intensity-projections).

The myocardium, respectively, is rendered relative to its overall median value, in order to cancel out contrast levels varying both between patients and exams.

The reader can interactively adjust certain Hounsfield unit (HU) based Window/Level settings, as well as select certain spectral maps of selective dual energy / photon counting energies (virtual mono-energy, iodine/electron density maps, etc).

Alternatively, a direct volume rendering is applied. Depth exploration can be provided by translucent volume rendering which visualizes also embedded anomalies. This technique does not require explicit segmentation nor centerline estimation.

In an embodiment, for any selected mapping, each map pixel is exported with its properties into a list of comma-separated values (e.g., one line per map pixel). In particular, each map pixel is exported with a differential area element, and a thickness element. Using these differential elements, numerical correlations etc. (between marked anomalies and coronary as well as myocardial properties) can be computed by a statistics program, without bias by the non-conformal mapping.

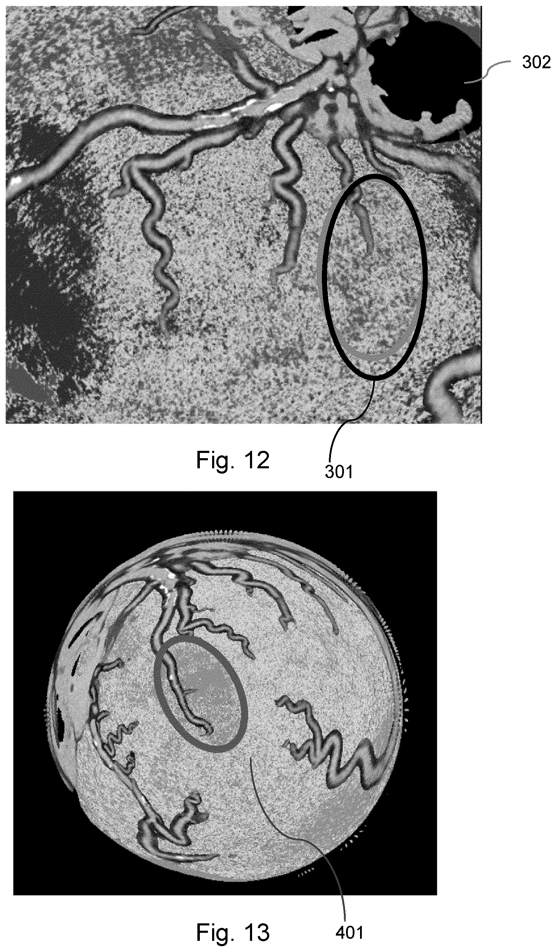

Fig. 12 shows and adjusted zoomed-in view, wherein the zoom factor has been adjusted by means of an interactive user interface (e.g. a slider). The zoomed-in view disloses HU-values of coronaries and color-scale-mapped median myocardial density, and a manual marking 301 of a (hypothetical) anomaly region as well as the cardiac atrium 302.

Fig. 13 shows an adjusted near-apical focused view, where the focus direction vector has been adjusted by means of an interactive user interface. A manual marking 401 of a coronary calcification and a (hypothetically) under-perfused myocardial area is also disclosed.

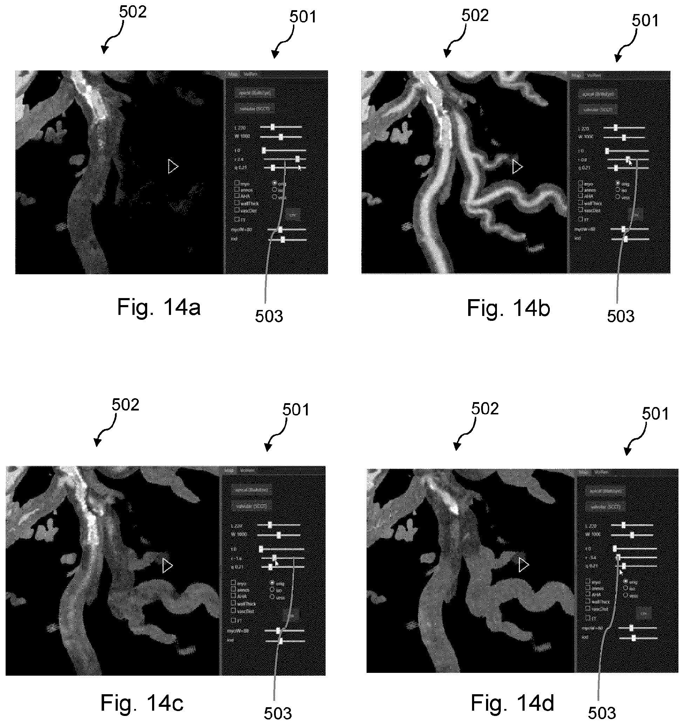

Fig. 14a, 14b, 14c and 14d show four views of a relative radial scrolling to inspect the local vascular depth exhaustively. The view shows an interactive user interface 501 next to a displaying view 502.

Each view represents a different intersection depth or local vascular depth. The intersection depth is adjusted by a slider 503 indicated by r (value for *Δr*). In this example, the intersection depth has a value of respectively +2.4, 0.0, -1.6 and -3.4.

By selecting or adjusting the intersection depth a relative radial scrolling is allowed to inspect a local vascular depth.

It will be appreciated that the system 10 for processing medical image data can be implemented in different forms, including but not limited to a workstation, a server, a distributed processing system, or a cloud-based system.

The solutions described herein can for example be used for interactive diagnostic appraisal on imaging workstations, as well as on devices with limited processing power including so-called thin clients, bed-side monitors, as well as 2D result-oriented PACS-workstations, and cohort- or follow-up-dashboards and structured reportings such as CAD-RADS.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. System (10) for processing medical image data for generating an image comprising a simultaneous representation of an arterial, valvular and myocardial state, wherein the system is adapted to perform the steps of:
- obtaining 3D medical image data (S101);
- identifying arterial, valvular and myocardial regions in the 3D medical image data by means of segmentation (S102);
- mapping the 3D medical image data to a 2D representation using a coordinate transform based on a spherical coordinate system with an origin depending on the identified valvular region and/or the identified arterial region (S103);
- generating a combined 2D image (50) indicative of the arterial-, valvular and myocardial state based on the 2D representation as an output (S104).

2. System according to claim 1, wherein the mapping of the 3D medical image data to a 2D representation is based on a spherical coordinate transform with an origin at a ventricle, in particular with an origin at a centroid of the left ventricle.

3. System according to claim 2, wherein the spherical coordinate transform is performed with a z-normal towards the aortic valve for generating a valvular view (51).

4. System according to claim 2, wherein the spherical coordinate transform is performed with a z-normal towards a ventricular apex, in particular towards the left ventricular apex, for generating and apical view (52).

5. System according to any of the preceding claims, wherein a volume of interest around the segmented valvular region (61) is mapped using the coordinate transform and using an intensity projection; in particular wherein for the valvular region (61) a mean intensity projection is used.

6. System according to any of the preceding claims, wherein 3D voxels of the segmented arteries of an arterial region (62) and/or 3D voxels of the segmented myocardial region (63) are mapped using the coordinate transform and using an intensity projection, wherein particularly for the arterial region (62) a maximum intensity projection is used and/or wherein for the myocardial region (63) a median intensity projection is used.

7. System according to claim 6, wherein the system is adapted to perform the steps of:
- determining a global median intensity of the myocardial region;
- determining a local median intensity for pixels of the 2D representation based on a median intensity projection;
- determining differences between the local median intensity and the global median for the pixels of the 2D representation; and
- generating a color-coded 2D representation indicative of the myocardial state based on the determined differences for the pixels of the 2D representation.

8. System according to any of the preceding claims, wherein for one or more of the pixels of the combined 2D image (50) a pointer to a corresponding location in the 3D medical image data is stored.

9. System according to any of the preceding claims, wherein mapping the 3D medical image data comprises:
- mapping the 3D medical image data to a first 2D representation (51) using a first coordinate transform based on a first spherical coordinate system with origin depending on the identified valvular region; and
- mapping the 3D medical image data to a second 2D representation (52) using a second coordinate transform based on a second spherical coordinate system with origin depending on the identified arterial region;
- wherein generating the combined 2D image (50) indicative of the arterial-, valvular and myocardial state comprises generating a first combined image part based on the first 2D representation (51) and a second combined image part the second 2D representation (52).

10. System according to claim 9, wherein the first combined image part (51) and the second combined image part (52) are arranged, in particular next to each other, in a single combined 2D image (50) indicative of the arterial-, valvular and myocardial state.

11. System according to any of the preceding claims, wherein the system is further adapted to perform a step of estimating myocardial feeding territories (65) and wherein the step of generating the combined 2D image (50) further comprises the estimated myocardial feeding territories.

12. System according to any of the preceding claims, wherein the system is further adapted to perform the step of mapping by means of a step of interactively adjusting a focus direction vector and/or a zoom factor using an interactive user interface, wherein the mapping depends on the adjusted focus direction vector and/or the zoom factor.

13. System according to any of the preceding claims, wherein the system is further adapted to perform the step of mapping by means of a step of interactively adjusting an intersection depth using an interactive user interface allowing adjustment of an intersection depth offset radially to the origin, wherein the offset is global and relative to a locally variable absolute 3D vector, wherein the mapping depends on the adjusted intersection depth.

14. Method (100) for processing medical image data for generating an image comprising a simultaneous representation of an arterial, valvular and myocardial state, the method comprising the steps of:
- obtaining 3D medical image data (S101);
- identifying arterial, valvular and myocardial regions in the 3D medical image data by means of segmentation (S102);
- mapping the 3D medical image data to a 2D representation using a coordinate transform based on a spherical coordinate system with an origin depending on the identified valvular region and/or the identified arterial region (S103);
- generating a combined 2D image (50) indicative of the arterial-, valvular and myocardial state based on the 2D representation as an output (S104).

15. Computer program comprising program code means for causing a computer to carry out the steps of the method (100) as claimed in claim 14 when said computer program is carried out on a computer.
